# EUROPEAN PATENT APPLICATION

(11) **EP 1 688 157 A1**
(43) Date of publication of application: **09.08.2006**
(21) Application number: 04819328.8
(22) Date of filing: 18.11.2004
(51) Int. Cl.: A61M 5/14, A61B 5/05, G01N 24/00

(54) **MEDICINE INFUSER**

(30) Priority: 25.11.2003 JP 2003393723
(71) Applicant: Nemoto Kyorindo Co., Ltd., Tokyo 113-0033 (JP)
(72) Inventor: NEMOTO, Shigeru c/o Nemoto Kyorindo Co., Ltd., Bunkyo-ku, Tokyo 1130033 (JP); YAZAWA, Akio c/o Nemoto Kyorindo Co., Ltd., Bunkyo-ku, Tokyo 1130033 (JP)
(74) Representative: Nargolwalla, Cyra
(86) International application number: PCT/JP2004/017190
(87) International publication number: WO 2005/051463

(57) **Abstract**

A wave emitting element emits a light beam to the predetermined position on the outer surface of the cylinder member of the liquid syringe at the predetermined angle, and the light beam is detected at the predetermined position on the outer surface of the cylinder member by a wave detecting element. When the light beam is detected, occurrence of abnormality is determined. Since the position on the cylinder member from which the light beam is directed when the liquid fills the cylinder member is different from that when air is trapped into or fills the cylinder member due to a change in refractive index, the occurrence of abnormality is determined and the check alarm is output when air is trapped into or fills the cylinder member. Thus, a chemical liquid injector which can detect and alarm air, if injected, when the liquid is injected into the patient from the liquid syringe through the extension tube is provided.

## Description

### Technical Field

The present invention relates to a chemical liquid injector for injecting a chemical liquid into a patient, and more particularly to a chemical liquid injector for injecting a contrast media into a patient who is to be imaged by an diagnostic imaging apparatus such as a CT (Computed Tomography) scanner, an MRI (Magnetic Resonance Imaging) apparatus, and a PET (Positron Emission Tomography) apparatus.

### Background Art

Presently, available diagnostic imaging apparatuses for imaging fluoroscopic images of patients include CT scanners, MRI apparatuses, PET apparatuses, ultrasonic diagnostic apparatuses, CT angiography apparatuses, and MR angiography apparatuses. When such a diagnostic imaging apparatus is used, a liquid such as a contrast media or physiological saline may be injected into the patient. Chemical liquid injectors for automatically performing the injection have been put into practical use.

Such a liquid injector has a drive motor and a slider mechanism and the like, and employs a liquid syringe which is removably mounted, for example. The liquid syringe comprises a cylinder member and a piston member slidably inserted in the cylinder member. The cylinder member is filled with a liquid such as a contrast media or physiological saline.

The liquid syringe is connected to the patient through an extension tube and set on a piston driving mechanism. The liquid injector individually holds the piston member and the cylinder member with the piston driving mechanism and moves them relatively to inject the liquid, typically the contrast media, from the liquid syringe into the patient.

An operator determines the rate at which the contrast media is injected and the total quantity of the contrast media to be injected, in view of various conditions, and then enters numerical data representing the rate and total quantity into the chemical liquid injector. The chemical liquid injector injects the contrast media into the patient at the rate and in the quantity represented by the entered numerical data. The injected contrast media changes the image contrast of the patient, allowing the imaging diagnostic apparatus to capture a good fluoroscopic image of the patient.

Some chemical liquid injectors are capable of injecting the physiological saline as well as the contrast media into the patient. In such a chemical liquid injector, a contrast media syringe filled with the contrast media and a physiological saline syringe filled with the physiological saline are mounted side by side as liquid syringes and are typically connected to the patient through a single bifurcated extension tube.

The operator enters, as desired, an instruction to inject the physiological saline following the completion of the injection of the contrast media, together with data representing the injection rate and total quantity of the physiological saline, into the chemical liquid injector. Based on the entered data, the chemical liquid injector first injects the contrast media into the patient and then automatically injects the physiological saline. The subsequently injected physiological saline can push the previously injected contrast media to reduce the consumption of the contrast media, and also can reduce artifacts in the captured image.

Chemical liquid injectors of the type described above have been devised and applied for patent by the applicant of the present application and the like (see, for example, patent documents 1 and 2 below).
Patent document 1: Japanese laid-open patent publication No. 2002-11096;
Patent document 2: Japanese laid-open patent publication No. 2002-102343.

### Disclosure of the Invention

### Subject to Be solved by the Invention

In the chemical liquid injector as described above, the liquid can be injected into the patient from the liquid syringe through the extension tube, and the contract media can be injected into a patient who is to be imaged by an imaging diagnostic apparatus, for example.

In injection of the liquid from the liquid syringe into the patient through a tube member in the chemical liquid injector, air trapped in the liquid causes a serious problem. In addition, a significant problem also arises if air is injected into the patient from the liquid syringe which is not filled with a liquid due to an operating error.

The present invention has been made in view of the abovementioned problems, and it is an object thereof to provide a chemical liquid injector which allows detection and notification of injection of air in injecting a liquid from a liquid syringe into a patient through an extension tube.

### Means to Solve the Subject

The present invention provides a chemical liquid injector for injecting a liquid from a liquid syringe including a cylinder member and a piston member slidably inserted into the cylinder member into a patient through an extension tube. According to a first and a second aspects of the present invention, the chemical liquid injector includes a cylinder holding mechanism, a piston driving means, a wave emitting element, a wave detecting element, an abnormality determining means, and an alarm notifying means.

In the chemical liquid injector according to the first aspect of the present invention, the cylinder holding mechanism removably holds the cylinder member of the liquid syringe, and the piston driving mechanism moves the piston member relative to the held cylinder member. The wave emitting element emits a wave to a predetermined position on an outer surface of the held cylinder member at a predetermined angle, and the wave detecting element detects the intensity of the wave at a predetermined position on the outer surface of the held cylinder member. The abnormality determining means determines occurrence of abnormality if the detected intensity of the wave does not comply with a predetermined allowable range, and the alarm notifying means outputs a check alarm when the occurrence of abnormality is determined.

Thus, in the chemical liquid injector according to the first aspect of the present invention, the wave is emitted to the predetermined position on the outer surface of the cylinder member of the liquid syringe at the predetermined angle, and the intensity of the wave is detected at the predetermined position on the outer surface of the cylinder member. Since the detected intensity of the wave when the liquid fills the cylinder member is different from that when air is trapped into or fills the cylinder member, the occurrence of abnormality is determined and the check alarm is output if air is trapped into or fills the cylinder member.

In the chemical liquid injector according to the second aspect of the present invention, the wave emitting element emits a wave to a predetermined position on an outer surface of the extension tube, and the wave detecting element detects the intensity of the wave at a predetermined position on the outer surface of the extension tube.

Thus, in the chemical liquid injector according to the second aspect of the present invention, the wave is emitted to the predetermined position on the outer surface of the extension tube at the predetermined angle, and the intensity of the wave is detected at the predetermined position on the outer surface of the extension tube. Since the detected intensity of the wave when the liquid fills the extension tube is different from that when air is trapped into or fills the extension tube, the occurrence of abnormality is determined and the check alarm is output when air is trapped into or fills the extension tube.

According to a third and a fourth aspects of the present invention, the chemical liquid injector includes a cylinder holding mechanism, a piston driving mechanism, a wave emitting element, a wave detecting element, a time measuring means, an abnormality determining means, and an alarm notifying means. In the chemical liquid injector according to the third aspect of the present invention, the wave emitting element emits a wave to a predetermined position on an outer surface of the held cylinder member at a predetermined angle, and the wave detecting element detects the wave at a predetermined position on the outer surface of the held cylinder member. The time measuring means measures a time period from the emission to the detection of the wave, and the abnormality determining means determines occurrence of abnormality if the measured time period does not comply with a predetermined allowable range.

Thus, in the chemical liquid injector according to the third aspect of the present invention, the wave is emitted to the predetermined position on the outer surface of the cylinder member of the liquid syringe at the predetermined angle, the wave is detected at the predetermined position on the outer surface of the cylinder member, and the time period from the emission to the detection is measured. Since the measured time period when the liquid fills the cylinder member is different from that when air is trapped into or fills the cylinder member, the occurrence of abnormality is determined and the check alarm is output if air is trapped into or fills the cylinder member.

In the chemical liquid injector according to the fourth aspect of the present invention, the wave emitting element emits a wave to a predetermined position on an outer surface of the extension tube at a predetermined angle, and the wave detecting element detects the wave at a predetermined position on the outer surface of the extension tube.

Thus, in the chemical liquid injector according to the fourth aspect of the present invention, the wave is emitted to the predetermined position on the outer surface of the extension tube at the predetermined angle, the wave is detected at the predetermined position on the outer surface of the extension tube, and the time period from the emission to the detection is measured. Since the measured time period when the liquid fills the extension tube is different from that when air is trapped into or fills the extension tube, the occurrence of abnormality is determined and the check alarm is output if air is trapped into or fills the extension tube.

According to a fifth and a sixth aspects of the present invention, the chemical liquid injector includes a cylinder holding mechanism, a piston driving mechanism, a wave emitting element, a wave detecting element, an abnormality determining means, and an alarm notifying means. In the chemical liquid injector according to the fifth aspect of the present invention, the wave emitting element emits light beam to a predetermined position on an outer surface of the held cylinder member at a predetermined angle. The wave detecting element detects the light beam at a predetermined position on the outer surface of the held cylinder member. The abnormality determining means determines occurrence of abnormality when the light beam is detected.

Thus, in the chemical liquid injector according to the fifth aspect of the present invention, the light beam is emitted to the predetermined position on the outer surface of the cylinder member of the liquid syringe at the predetermined angle, and the light beam is detected at the predetermined position on the outer surface of the cylinder member. Since the position on the cylinder member from which the light beam is directed when the liquid fills the cylinder member is different from that when air is trapped into or fills the cylinder member due to a change in refractive index, the occurrence of abnormality is determined and the check alarm is output when air is trapped into or fills the cylinder member.

In the chemical liquid injector according to the sixth aspect of the present invention, the wave emitting element emits light beam to a predetermined position on an outer surface of the extension tube at a predetermined angle, and the wave detecting element detects the light beam at a predetermined position on the outer surface of the extension tube.

Thus, in the chemical liquid injector according to the sixth aspect of the present invention, the light beam is emitted to the predetermined position on the outer surface of the extension tube at the predetermined angle, and the light beam is detected at the predetermined position on the outer surface of the extension tube. Since the position on the cylinder member from which the light beam is directed when the liquid fills the extension tube is different from that when air is trapped into or fills the extension tube due to a change in refractive index, the occurrence of abnormality is determined and the check alarm is output when air is trapped into or fills the extension tube.

According to a seventh and an eighth aspects of the present invention, the chemical liquid injector includes a cylinder holding mechanism, a piston driving mechanism, a wave emitting element, a wave detecting element, a characteristic analyzing means, an abnormality determining means, and an alarm notifying means. In the chemical liquid injector according to the seventh aspect of the present invention, the wave emitting element emits an ultrasonic wave to a predetermined position on an outer surface of the held cylinder member at a predetermined angle. The wave detecting element detects the ultrasonic wave at a predetermined position on the outer surface of the held cylinder member. The characteristic analyzing means analyzes the resonance characteristic of the detected ultrasonic wave, and the abnormality determining means determines occurrence of abnormality from the analyzed resonance characteristic.

Thus, in the chemical liquid injector according to the seventh aspect of the present invention, the ultrasonic wave is emitted to the predetermined position on the outer surface of the cylinder member of the liquid syringe at the predetermined angle, and the ultrasonic wave is detected at the predetermined position on the outer surface of the cylinder member. Since the resonance characteristic when the liquid fills the cylinder member is different from that when air is trapped into or fills the cylinder member, the occurrence of abnormality is determined and the check alarm is output when air is trapped into or fills the cylinder member.

In the chemical liquid injector according to the eighth aspect of the present invention, the wave emitting element emits an ultrasonic wave to a predetermined position on an outer surface of the extension tube at a predetermined angle, and the wave detecting element detects the ultrasonic wave at a predetermined position on the outer surface of the extension tube.

Thus, in the chemical liquid injector according to the eighth aspect of the present invention, the ultrasonic wave is emitted to the predetermined position on the outer surface of the extension tube at the predetermined angle, the ultrasonic wave is detected at the predetermined position on the outer surface of the extension tube, and the resonance characteristic of the detected ultrasonic wave is analyzed. Since the resonance characteristic when the liquid fills the extension tube is different from that when air is trapped into or fills the extension tube, the occurrence of abnormality is determined and the check alarm is output when air is trapped into or fills the extension tube.

According to a ninth and a tenth aspects of the present invention, the chemical liquid injector includes a cylinder holding mechanism, a piston driving mechanism, a capacitance detecting sensor, an abnormality determining means, and an alarm notifying means. In the chemical liquid injector according to the ninth aspect of the present invention, the capacitance detecting sensor detects a capacitance on an outer surface of the held cylinder member, and the abnormality determining means determines occurrence of abnormality if the detected capacitance does not comply with a predetermined allowable range.

Thus, in the chemical liquid injector according to the ninth aspect of the present invention, the capacitance is detected on the outer surface of the cylinder member of the liquid syringe. Since the capacitance when the liquid fills the cylinder member is different from that when air is trapped into or fills the cylinder member, the occurrence of abnormality is determined and the check alarm is output when air is trapped into or fills the cylinder member.

In the chemical liquid injector according to the tenth aspect of the present invention, a wave emitting element emits light beam to a predetermined position on an outer surface of the extension tube at a predetermined angle, and a wave detecting element detects the light beam at a predetermined position on the outer surface of the extension tube.

Thus, in the chemical liquid injector according to the tenth aspect of the present invention, the capacitance is detected on the outer surface of the extension tube by the capacitance detecting sensor. Since the capacitance when the liquid fills the extension tube is different from that when air is trapped into or fills the extension tube, the occurrence of abnormality is determined and the check alarm is output when air is trapped into or fills the extension tube.

The various means referred to in the present invention may be arranged to perform their prescribed functions, and may be implemented by dedicated pieces of hardware for performing the functions, data processing apparatus for performing the functions according to computer programs, functions achieved in data processing apparatus according to computer programs, or combinations thereof.

The various means referred to in the present invention are not required to be individually independent entities, and may be arranged such that a plurality of components may be constructed as a single member, a certain component may be part of another component, or part of a certain component and part of another component overlap each other.

### Effect of the Invention

In the chemical liquid injector according to the present invention, the occurrence of abnormality is determined and the check alarm is output when air is trapped into or fills the cylinder member or the extension tube, so that the operator can quickly recognize air, if injected, when the liquid is injected into the patient from the liquid syringe through the extension tube.

### Brief Description of the Drawings

Fig. 1 is a schematic diagram showing the logical structure of a chemical liquid injecting system according to Embodiment 1 of the present invention.
Fig. 2 is a block diagram showing the physical structure of the chemical liquid injecting system.
Fig. 3 is a perspective view showing the exterior of the chemical liquid injecting system.
Fig. 4 is a perspective view showing the exterior of a chemical liquid injector of the chemical liquid injecting system.
Figs. 5(a) and 5(b) are perspective views how to mount a liquid syringe on an injection head of the chemical liquid injector.
Figs. 6(a) to 6(c) are cross-sectional views showing the relationship between an air detection unit and the liquid syringe.
Fig. 7 is a flow chart showing the processing operation of the chemical liquid injector.
Fig. 8 is a flow chart showing the processing operation of an MRI apparatus serving as an imaging diagnostic apparatus.
Figs. 9(a) to 9(c) are cross-sectional views showing the relationship between an air detection unit and a liquid syringe according to Embodiment 2.
Figs. 10(a) to 10(c) are cross-sectional views showing the relationship between an air detection unit and a liquid syringe according to a modification.
Fig. 11(a) to 11(c) are cross-sectional views showing the relationship between an air detection unit and a liquid syringe according to Embodiment 3.
Figs. 12(a) to 12(c) are cross-sectional views showing the relationship between an air detection unit and a liquid syringe according to Embodiment 4.

### Description of Reference Numerals

- 100: CHEMICAL LIQUID INJECTOR
- 115: CYLINDER HOLDING MECHANISM
- 116: PISTON DRIVING MECHANISM
- 121: MORVABLE ARM SERVING AS COMPONENT PLACING MECHANISM
- 123: LIGHT EMITTING ELEMENT SERVING AS WAVE EMITTING ELEMENT
- 124: LIGHT RECEIVING ELEMENT SERVING AS WAVE DETECTING ELEMENT
- 130: COMPUTER UNIT
- 141: ABNORMALITY DETERMINING MEANS
- 142: ALARM NOTIFYING MEANS
- 143: DRIVE STOPPING MEANS
- 144: STOP OUTPUTTING MEANS
- 200: LIQUID SYRINGE
- 210: CYLINDER MEMBER
- 220: PISTON MEMBER
- 230: EXTENSION TUBE
- 300: MRI APPARATUS SERVING AS IMAGING DIAGNOSTIC APPARATUS
- 301: DIAGNOSTIC IMAGING UNIT
- 311: STOP INPUTTING MEANS
- 312: ALARM OUTPUTTING MEANS
- 313: IMAGING STOPPING MEANS
- 431: ULTRASONIC VIBRATOR SERVING AS WAVE EMITTING ELEMENT
- 432: ULTRASONIC DETECTOR SERVING AS WAVE DETECTING ELEMENT

### Best Mode for Carrying Out the Invention

### [Configuration of Embodiment 1]

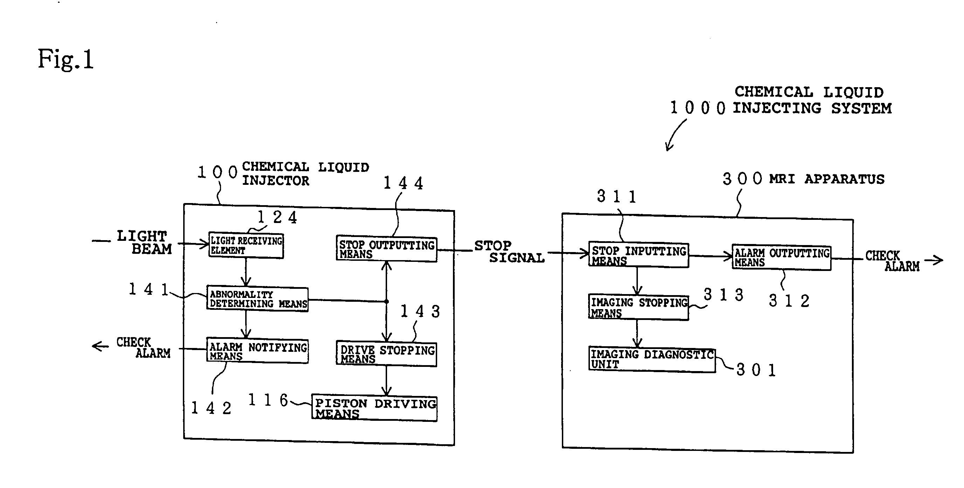

Embodiment 1 of the present invention will hereinafter be described with reference to Figs. 1 to 8. As shown in Figs. 1 to 3, chemical liquid injecting system 1000 of Embodiment 1 according to the present invention comprises chemical liquid injector 100, liquid syringe 200, and MRI apparatus 300 which is an imaging diagnostic apparatus. The system is intended for injecting a contrast media or the like as a chemical liquid into a patient (not shown), as later described in detail.

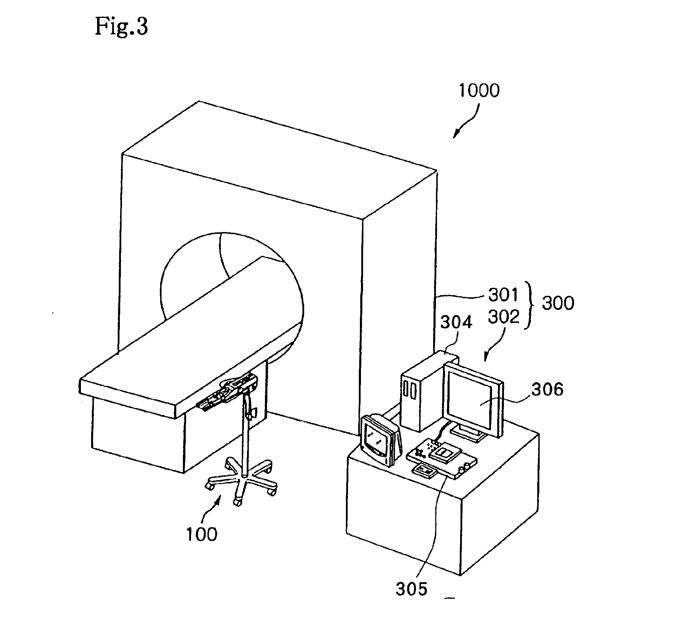

As shown in Fig. 3, MRI apparatus 300 includes diagnostic imaging unit 301 serving as a mechanism for performing imaging and imaging control unit 302 such that diagnostic imaging unit 301 and imaging control unit 302 are wire-connected through communication network 307. Diagnostic imaging unit 301 takes a diagnostic image of a patient. Imaging control unit 302 controls the operation of diagnostic imaging unit 301.

Imaging control unit 302 includes hardware similar to a personal computer and includes main control unit body 304 for performing various types of data processing, keyboard 305 for inputting various types of data, display 306 for outputting various types of data as display, a speaker unit (not shown) for outputting various types of data as sound, and the like.

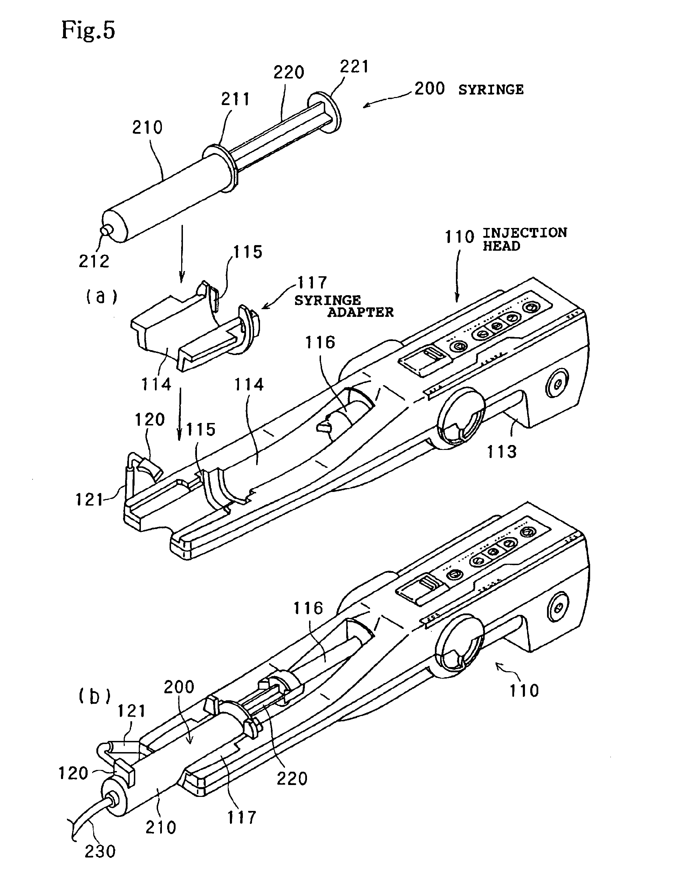

As shown in Fig. 5(a), liquid syringe 200 comprises cylinder member 210 and piston member 220 wherein piston member 220 is slidably inserted into cylinder member 210. Cylinder member 210 includes cylindrical hollow body 211 which has conduit tube 212 formed in the closed leading end surface thereof

The trailing end of body 211 of cylinder member 210 is opened and piston member 220 is inserted from the opening into the interior of body 211. Cylinder member 210 has cylinder flange 213 formed in the outer circumference of the trailing end, and piston member 220 has piston flange 221 formed in the outer circumference of the trailing end.

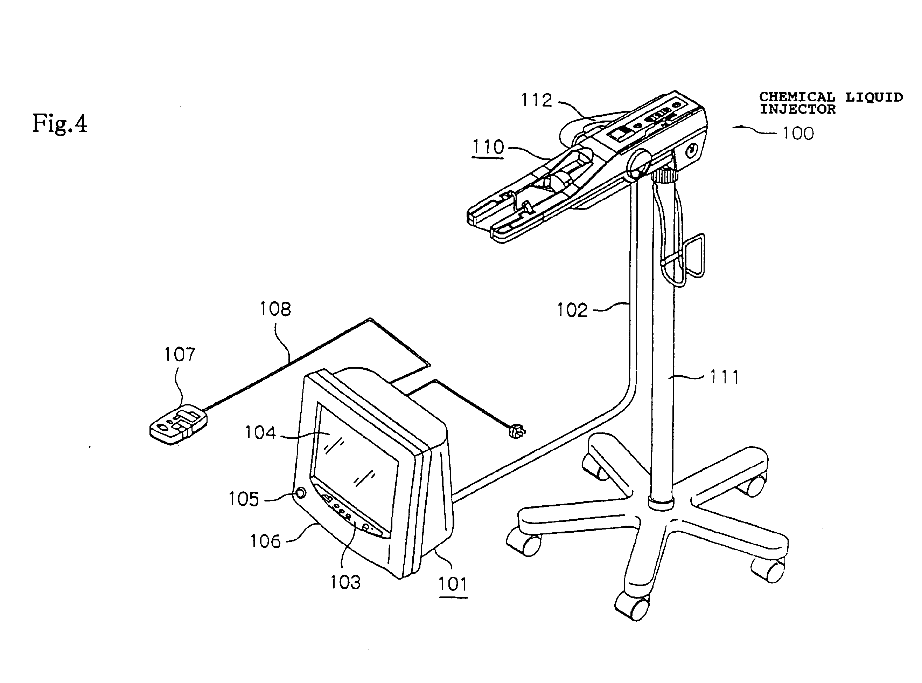

As shown in Fig. 4, chemical liquid injector 100 of Embodiment 1 has injection control unit 101 and injection head 110 constructed as separate components which are wire-connected through communication cable 102. Injection head 110 drives liquid syringe 200 mounted thereon to inject a liquid therefrom into a patient. Injection control unit 101 controls the operation of injection head 110.

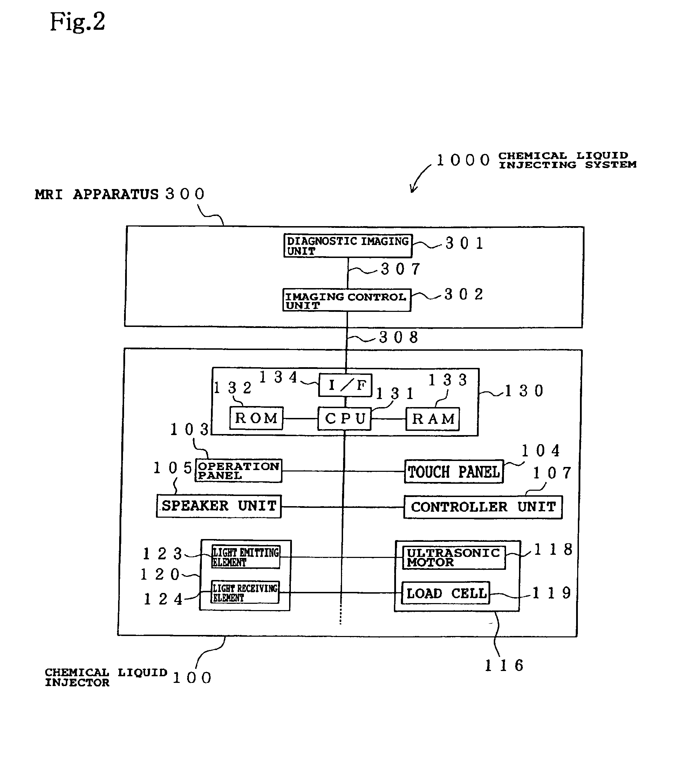

Thus, as shown in Fig. 2, injection control unit 101 contains computer unit 130 therein and is wire-connected to imaging control unit 302 of MRI apparatus 300 through communication network 308. Injection control unit 101 has operation panel 103, touch panel 104, and speaker unit 105, all of which are disposed on the front face of unit housing 106. Injection control unit 101 is wire-connected to controller unit 107 as a separate component through connector 108.

Injection head 110 is attached to the top end of caster stand 111 by movable arm 112. As shown in Fig. 4, head body 113 has concave portion 114 formed as semi-cylindrical groove in the upper surface for removably mounting liquid syringe 200.

Concave portion 114 has cylinder holding mechanism 115 formed in the forward section for removably holding cylinder flange 211 of liquid syringe 200 and also has piston driving mechanism 116 arranged in the rearward section for holding and sliding piston flange 221.

Cylinder holding mechanism 115 is formed as a groove having a different shape with concave portion 114, with which cylinder flanges 211 removably engages. Since various types of liquid syringes 200 are mounted on chemical liquid injector 100 in chemical liquid injecting system 1000 of the present embodiment, concave portion 114 and cylinder holding mechanism 115 of chemical liquid injector 100 is formed to accommodate liquid syringe 200 of the largest size.

Thus, in chemical liquid injecting system 1000 of the present embodiment, as shown in Figs. 5(a) and 5(b), cylinder adapter 117 is provided for each of liquid syringes 200 of sizes other than the largest size, and liquid syringe 200 of a size other than the largest size is mounted on chemical liquid injector 100 with cylinder adapter 117.

Piston driving mechanism 116 has ultrasonic motor 118 as a driving source which is free from generation of magnetic field even in operation, and slides piston member 220 through a screw mechanism (not shown) or the like. Load cell 118 is also contained in piston driving mechanism 116 and detects the pressure on piston member 220.

In chemical liquid injector 100 of the present embodiment, respective components of injection head 110 are made of nonmagnetic material, and the portions that cannot be made of nonmagnetic material are magnetically shielded. For example, ultrasonic motor 118 and load cell 119 are made of nonmagnetic metal such as phosphor bronze alloy (Cu + Sn +P), titanium alloy (Ti-6Al-4V), and magnesium alloy (Mg + Al +Zn). Head body 113 or the like is made of nonmagnetic resin.

In chemical liquid injector 100 of the present embodiment, air detection unit 120 is disposed near the leading end of injection head 110 and detects air inside cylinder member 210 of liquid syringe 200.

More particularly, air detection unit 120 is mounted on the leading end of movable arm 121 serving as a component placing mechanism. The trailing end of movable arm 121 is attached to the side of injection head 110 at the leading end thereof such that movable arm 121 is pivoted vertically. Liquid syringes 200 of various sizes are mounted on chemical liquid injector 100 as described above. Since movable arm 121 is extensible, air detection unit 120 is disposed such that it can be in contact or not in contact with the upper outer surface of liquid syringe 200 near the leading end thereof.

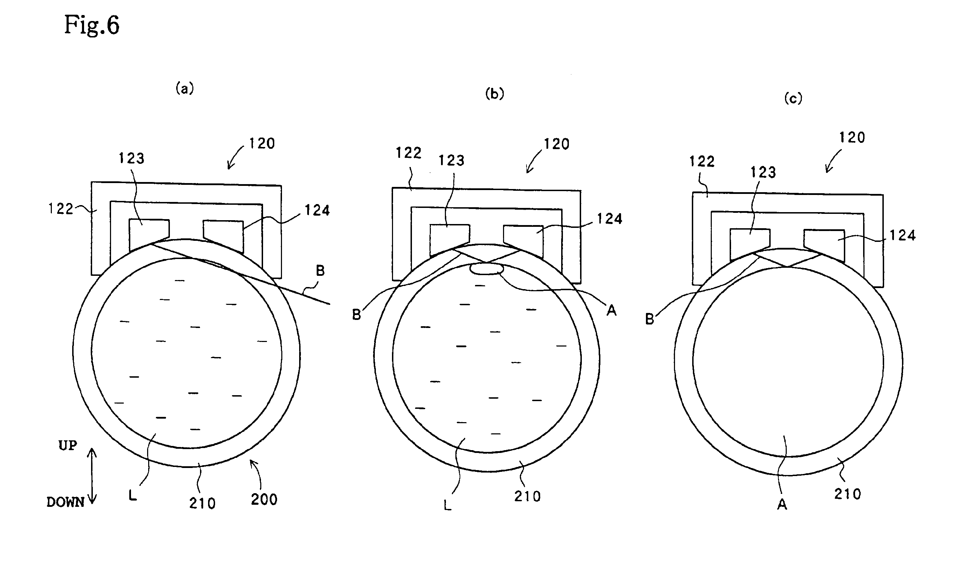

Air detection unit 120 has unit body 122 in box shape. As shown in Figs. 6(a) to 6(c), unit body 122 contains light emitting element 123 serving as a wave emitting element and a light receiving element 124 serving as a wave detecting element.

Light emitting element 123 comprises a laser diode or the like and emits light beam B as a wave to a predetermined position on the outer surface of cylinder member 210 of liquid syringe 200 at a predetermined angle. Light receiving element 124 comprises a photosensor or the like and detects light beam B at a predetermined position on the outer surface of cylinder member 210.

Cylinder member 210 of liquid syringe 200 is made of glass or transparent resin and has a refractive index which is greatly different from that of air A and is close to that of liquid L. Thus, when cylinder member 210 is filled with liquid L as shown in Fig. 6(a), light beam B emitted from light emitting element 123 travels generally straight without being reflected off the inner surface of cylinder member 210 or the like. However, as shown in Figs. 6(b) and 6(c), when air A exists in the path of light beam B emitted from light emitting element 123, light beam B is reflected off the inner surface of cylinder member 210.

Light emitting element 123 and light receiving element 124 are disposed in close contact with the outer surface of cylinder member 210 of liquid syringe 200 such that light beam B emitted from light emitting element 123 is reflected off the inner surface of cylinder member 210 and enters light receiving element 124.

light beam B emitted from light emitting element 123 is not detected by light receiving element 124 when cylinder member 210 is filled with liquid L as shown in Fig. 6(a). When air A exists inside cylinder member 210 as shown in Figs. 6(b) and 6(c), light beam B emitted from light emitting element 123 is detected by light receiving element 124.

As shown in Fig. 2, in chemical liquid injector 100 of the present embodiment, the abovementioned various devices are connected to computer unit 130 which integrates and controls those various devices. Computer unit 130 comprises a so-called one-chip microcomputer provided with hardware such as CPU (Central Processing Unit) 131, ROM (Read Only Memory) 132, RAM (Random Access Memory) 133, I/F (Interface) 134 and the like.

Computer unit 130 has an appropriate computer program installed as firmware or the like in an information storage medium such as ROM 132, and CPU 131 executes various types of processing in accordance with the computer program.

CPU 131 operates in accordance with the computer program installed as described above to allow chemical liquid injector 100 of the present embodiment to logically have, as various functions, various means including abnormality determining means 141, alarm notifying means 142, drive stopping means 143, and stop outputting means 144.

Similarly, main control unit 304 operates in accordance with the installed computer program to perform various types of operations to allow MRI apparatus 300 to logically have, as various functions, various means including stop inputting means 311, alarm outputting means 312, and imaging stopping means 313.

Abnormality determining means 141 of chemical liquid injector 100 corresponds to the function of CPU 131 recognizing data of a signal detected by light receiving element 124 of air detection unit 120 in accordance with the computer program installed in ROM 132 and the like, and determines the occurrence of abnormality if light receiving element 124 detects light beam B.

More particularly, when chemical liquid injector 100 drives liquid syringe 200 mounted on injection head 110 to inject liquid L into a patient, computer unit 130 drives light emitting element 123 of air detection unit 120 to emit bean light B. Consequently, when light receiving element 124 detects light beam B, computer unit 130 determines the occurrence of abnormality.

Alarm notifying means 142 corresponds to the function of CPU 131 outputting data stored in ROM 132 on touch panel 104 or from speaker unit 105 in accordance with the computer program, and outputs a check alarm when abnormality determining means 141 determines the occurrence of abnormality.

More particularly, ROM 132 has a guidance message as text data stored therein such as "Air detected in syringe. Check syringe." When light receiving element 124 of air detection unit 120 detects light beam B, CPU 131 receives the detection signal and reads out that guidance message from ROM 132 and outputs it as display on touch panel 104 and as sound from speaker unit 105.

Drive stopping means 143 corresponds to the function of CPU 131 controlling the operation of ultrasonic motor 118 of piston driving mechanism 116, and forcedly stops piston driving mechanism 116 when abnormality determining means 141 determines the occurrence of abnormality. Stop outputting means 144 corresponds to the function of CPU 131 outputting the data stored in ROM 132 to the outside from I/F 134 through communication network 308, and outputs a stop signal to the outside when abnormality determining means 141 determines the occurrence of abnormality.

Stop inputting means 311 of MRI apparatus 300 corresponds to the function of main control unit 304 of imaging control unit 302 recognizing the data of a signal input thereto through communication network 308 in accordance with the installed computer program, and receives a stop signal from chemical liquid injector 100.

Alarm outputting means 312 corresponds to the function of main control unit 304 outputting the stored data on display 306 and the speaker unit, and outputs the guidance message such as "Air detected in syringe. Check syringe" as an alarm when it receives a stop signal from chemical liquid injector 100.

Imaging stopping means 313 corresponds to the function of imaging control unit 302 controlling the operation of diagnostic imaging unit 301 and forcedly stops the imaging operation of diagnostic imaging unit 301 when a stop signal is input thereto from the outside.

While the above various means of chemical liquid injector 100 are accomplished by pieces of hardware such as touch panel 104 as required, they are mainly implemented by CPU 131 as a piece of hardware functioning in accordance with resources and computer programs stored on an information storage medium such as ROM 132.

Such computer programs are stored in an information storage medium such as RAM 133 as software for causing CPU 131 or the like to perform processing operations including determination of the occurrence of abnormality upon detection of light beam B by light receiving element 124 when light emitting element 123 of air detection unit 120 is driven, output of a check alarm as display output on touch panel 104 and sound output from speaker unit 105 when the occurrence of abnormality is determined, forced stop of piston driving mechanism 116 when the occurrence of abnormality is determined, and output of a stop signal to the outside from I/F 134 through communication network 308 when the occurrence of abnormality is determined.

Similarly, the computer programs installed in main control unit 304 of MRI apparatus 300 comprise software for causing main control unit 304 to perform processing operations including input of a stop signal from chemical liquid injector 100 through communication network 308, output of a check alarm on display 306 or the speaker unit when the stop signal is input from the outside, and forced stop of the imaging operation of diagnostic imaging unit 301 when the stop signal is input from the outside.

### [Operation of Embodiment 1]

For using chemical liquid injector 100 of Embodiment 1 in the above construction, an operator (not shown) disposes chemical liquid injector 100 near imaging unit 301 of MRI apparatus 300 as shown in Fig. 3, and prepares liquid syringe 200 and extension tube 230 for use.

The operator connects liquid syringe 200 to a patient (not shown) placed in imaging unit 301 through extension tube 230 and mounts liquid syringe 200 on injection head 110 of chemical liquid injector 100. If liquid syringe 200 is of the largest size, cylinder member 210 is inserted into concave portion 114 of injection head 110 from above to hold cylinder flange 211 in cylinder holding mechanism 115 and piston flange 221 in piston driving mechanism 116.

If liquid syringe 200 is not of the largest size, that liquid syringe 200 is mounted on injection head 110 with cylinder adapter 117 interposed between them as described above. When liquid syringe 200 is set in concave portion 114 of injection head 110 as described above, it is put in concave portion 114 from the direction in which it can avoid interfering with air detection unit 120 and movable arm 121 since air detection unit 120 is attached to injection head 110 by movable arm 121 above concave portion 114 as shown in Fig. 5(a).

After the operator puts liquid syringe 200 in concave portion 114 of injection head 110, he or she turns movable arm 121 forward and extends or retracts it to bring air detection unit 120 in close contact with the outer surface of cylinder member 210 of liquid syringe 200 near the leading end thereof from above.

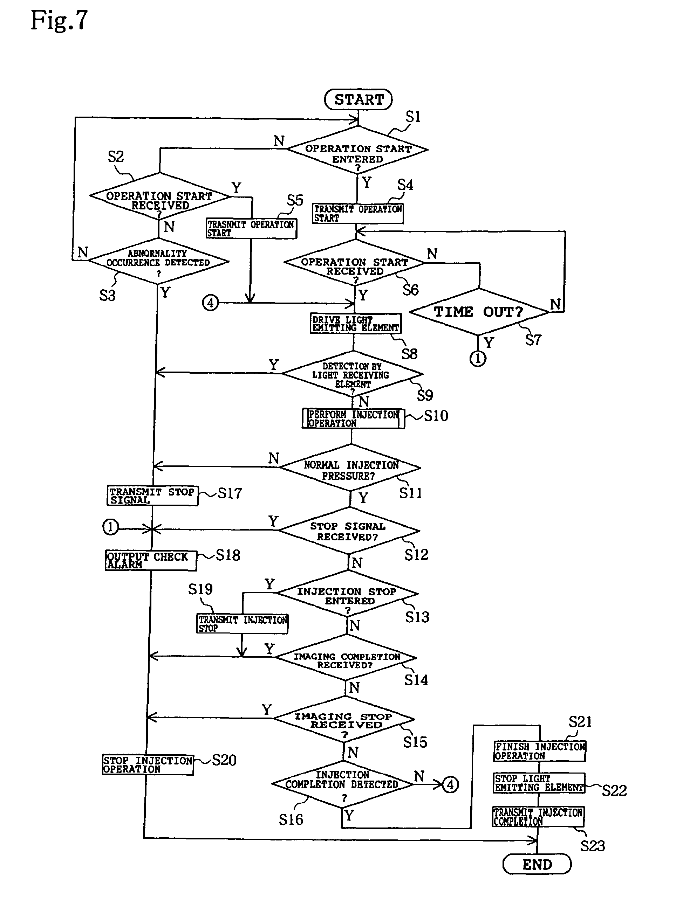

In this state, when the operator makes an input action to start operation on touch panel 104 or operation panel 103 of chemical liquid injector 100, chemical liquid injector 100 detects it (step S1) and transmits data to start operation to MRI apparatus 300 (step S4) as shown in Fig. 7.

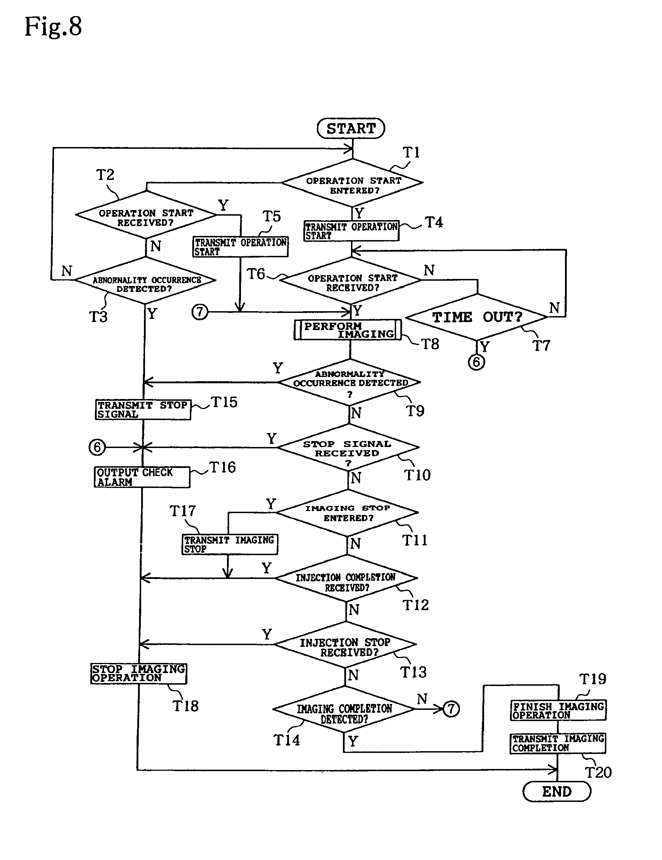

As shown in Fig. 8, MRI apparatus 300 receives the data to start operation from chemical liquid injector 100 (step T2) and transmits the data to start operation back to chemical liquid injector 100 to perform imaging operation (step T8). Thus, in chemical liquid injecting system 1000 of the present embodiment, the imaging of MRI apparatus 300 follows the liquid injection of chemical liquid injector 100.

In chemical liquid injecting system 1000 of the present embodiment, as shown in Figs. 7 and 8, if an operator makes an input action to start imaging on MRI apparatus 300 (step T1) when chemical liquid injector 100 is ready as described above (steps S1 - S3), the liquid injection of chemical liquid injector 100 follows the imaging of MRI apparatus 300 (steps T4, T6 and afterward, S2, S5, S8 and afterward).

As shown in Fig. 7, in chemical liquid injector 100 of the present embodiment, light emitting element 123 of air detection unit 120 is started first in liquid injection (step S8). When cylinder member 210 of liquid syringe 200 is filled only with liquid L as shown in Fig. 6(a), light beam B emitted from light emitting element 123 is not detected by light receiving element 124. When air A is trapped or filled into cylinder member 210, light beam B is detected by light receiving element 124.

If light receiving element 124 does not detect light beam B (step S9), computer unit 130 determines no occurrence of abnormality of air A present inside cylinder member 210, and piston driving mechanism 116 is driven to inject liquid L from liquid syringe 200 into the patient (step S10).

On the other hand, if light receiving element 124 detects light beam B (step S9), computer unit 130 determines the occurrence of abnormality of air A present inside cylinder member 210. In this case, since piston driving mechanism 116 is forcedly stopped (step S20), air A is not injected from liquid syringe 200 into the patient.

In addition, the guidance message such as "Air detected in syringe. Check syringe" is output as display on touch panel 104 and output as sound from speaker unit 105 as the check alarm (step S18). The operator quickly recognizes and deals with the abnormality of air A present inside liquid syringe 200.

Since the stop signal is transmitted from chemical liquid injector 200 to MRI apparatus 300 (step S17), MRI apparatus 300 receives the stop signal (step T10) and forcedly stops the imaging operation of diagnostic imaging unit 301 (step T18). In addition, since MRI apparatus 300 also outputs the alarm (step T16), the operator quickly recognizes and deals with the abnormality of air A present inside liquid syringe 200 even when he or she is located away from chemical liquid injector 100 and near MRI apparatus 300.

In chemical liquid injector 100 and MRI apparatus 300 of the present embodiment, when the occurrence of abnormality is detected in the ready state described above (steps S3, T3) or when MRI apparatus 300 detects the occurrence of abnormality during the imaging operation (step T9), then the occurrence of abnormality is notified (steps S18, T16) and their operations are stopped (steps S20, T18).

When an input action to stop the operation is made in one apparatus (steps S13, T11), the operation is stopped in the apparatus (steps S20, T18) and that is notified to the other apparatus (steps S19, T17). As a result, the other apparatus which has received that notification (steps T13, S15) stops its operation (steps T18, S20)**.**

Furthermore, when the completion of the operation is detected in one apparatus (steps S16, T14), the operation is finished in the apparatus (steps S21, T19) and that is notified to other apparatus (steps S23, T20). As a result, the other apparatus which has received the notification (steps T12, S14) stops its operation (steps T18, S20).

### [Effect of Embodiment 1]

In chemical liquid injector system 1000 of Embodiment 1, liquid L such as a contract media or physiological saline is injected from liquid syringe 200 of chemical liquid injector 100 into the patient which is to be imaged by MRI apparatus 300, and when air A is trapped or filled into liquid syringe 200, chemical liquid injector 100 determines the occurrence of abnormality.

When the occurrence of abnormality is detected in this manner, piston driving mechanism 116 is forcedly stopped, thereby making it possible to automatically prevent erroneous operation of injection of air A into the patient from liquid syringe 200. In addition, since the alarm is notified as display output on touch panel 104 and sound output from speaker unit 105, the operator quickly recognizes and deals with the abnormality of air A present inside liquid syringe 200.

Since the stop signal is transmitted from chemical liquid injector 100 to MRI apparatus 300, MRI apparatus 300 forcedly stops the imaging operation of diagnostic imaging unit 301. This can prevent unnecessary imaging operation and facilitate the check of liquid syringe 200 by the operator.

MRI apparatus 300 which has received the stop signal also outputs the alarm, so that the operator can quickly recognize and deal with the abnormality of air A present inside liquid syringe 200 even when he or she is located away from chemical liquid injector 100 and near MRI apparatus 300.

In chemical liquid injecting system 1000 of the present embodiment, as shown in Figs. 6(a) to 6(c), light emitting element 123 emits light beam B to the predetermined position on the outer surface of cylinder member 210 of liquid syringe 200 at the predetermined angle, and light receiving element 124 detects light beam B at the predetermined position on the outer surface of cylinder member 210 to detect the state inside cylinder member 210 such as trapping or filling of air A. As a result, trapping of air A can be detected favorably with a simple structure.

Particularly, since light emitting element 123 and light receiving element 124 are formed integrally with unit body 122, light emitting element 123 and light receiving element 124 can be disposed at appropriate angles and positions relative to the outer surface of cylinder member 210.

In chemical liquid injecting system 1000 of the present embodiment, a plurality of types of liquid syringes 200 having cylinder members 210 with different outer diameters are used. Since air detection unit 120 is supported movably vertically by movable arm 121 and is disposed on the outer surface of cylinder member 210, air detection unit 120 can be appropriately positioned on the outer surfaces of the plurality of types of cylinder members 210 with different outer diameters.

If a small amount of air A is trapped in liquid L inside liquid syringe 200 as shown in Fig. 6(b), that air A is located in an upper portion of the interior of cylinder member 210. Since air detection unit 120 is disposed on the outer surface of cylinder member 210 from above in chemical liquid injecting system 1000 of the present embodiment, even the small amount of air A can be detected favorably.

When piston member 220 inserted into cylinder member 210 moves to the position where air detection unit 120 can detect it, piston member 220 may be detected erroneously as air. However, in chemical liquid injecting system 1000 of the present embodiment, movable arm 121 is extensible and thus air detection unit 120 can be disposed near the leading end of cylinder member 210 to prevent unnecessary, erroneous detection of piston member 220.

### [Modifications of Embodiment 1]

The present invention is not in any way limited to the abovementioned embodiment, but various changes or modifications may be made without departing from the scope of the invention. For example, although air detection unit 120 supported movably by movable arm 121 is disposed on the outer surface of cylinder member 210 of liquid syringe 200 in the above embodiment, it may be disposed on the outer surface of extension tube 230 (not shown).

In this case, the air detection unit is preferably reduced in size as appropriate for use with extension tube 230, wire-connected to injection head 110, and provided integrally with a tool such as a clothespin which is used for mounting on the outer surface of extension tube 230.

In the above-described embodiment, the occurrence of abnormality is determined without considering the type of liquid syringe 200 or the like by chemical liquid injector 100. However, when a plurality of types of liquid syringes 200 are used with different cylinder members 210 or different liquids L with different physical properties, the type of liquid syringe 200 may be input to operation panel 103 or touch panel 104 to allow computer unit 130 to determine the occurrence of abnormality based on the type. In this case, even when the plurality of types of liquid syringes 200 have different cylinder members 210 or use liquids L with different physical properties, the occurrence of abnormality can be determined satisfactorily in accordance with the physical properties.

In the above-described embodiment, chemical liquid injector 100 injects a single type of liquid L such as the contrast media with the single piston driving mechanism 116. However, it is possible to realize a chemical liquid injector which uses two piston driving mechanisms 116 to flexibly inject a contrast media and physiological saline or a chemical liquid injector which uses three or more piston driving mechanisms 116 to inject three or more types of liquid L (not shown).

In the above-described embodiment, MRI apparatus 300 is used as the diagnostic imaging apparatus, and chemical liquid injector 100 injects the contrast media for use in the MRI apparatus. A CT scanner or a PET apparatus may be used as the diagnostic imaging apparatus and the chemical liquid injector may inject a contrast media for use in such an apparatus.

In the above-described embodiment, CPU 131 operates in accordance with the computer program stored in RAM 133 or the like to logically perform the various means as the various functions of chemical liquid injector 100. The above means may be implemented by pieces of hardware, or some of the means may be stored as software in RAM 133 or the like and the others implemented by pieces of hardware.

### [Configuration of Embodiment 2]

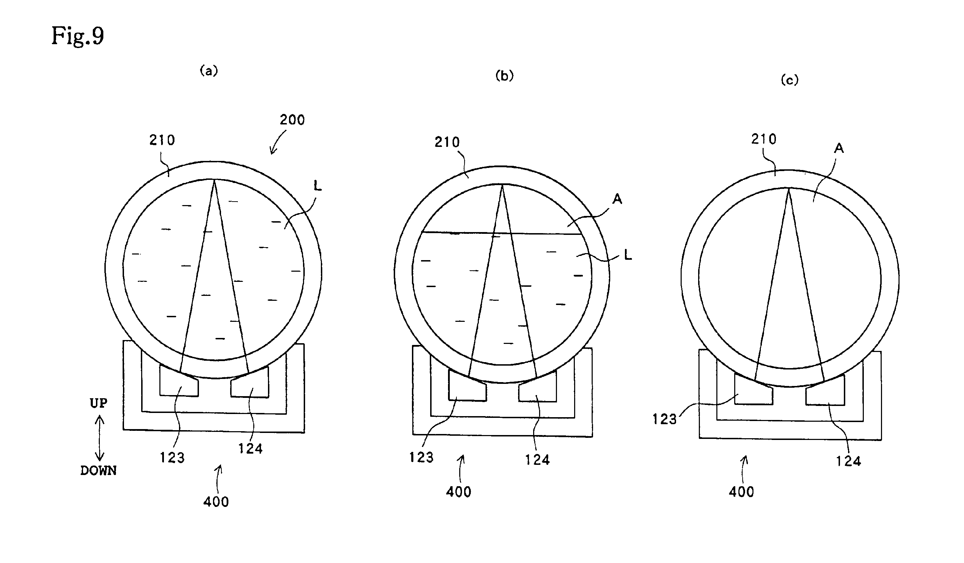

Next, Embodiment 2 of the present invention will hereinafter be described with reference to Figs. 9(a) to 9(c). In the present and following embodiments, components identical to those in Embodiment 1 described above are designated with the same name and reference numerals, and detailed description thereof are omitted.

In a chemical liquid injector (not shown) of the present embodiment, air detection unit 400 includes light emitting element 123 and light receiving element 124 just the same as Embodiment 1. However, air detection unit 400 is disposed by movable arm 121 such that it can be in contact or not in contact with the outer surface of cylinder member 210 of liquid syringe 200 near the leading end thereof from below.

Light emitting element 123 of air detection unit 400 emits light beam B as a wave upward to a predetermined position on the lower outer surface of cylinder member 210 at a predetermined angle. Light receiving element 124 detects light beam B entering it from above at a predetermined of the lower outer surface of cylinder member 210. Light emitting element 123 is disposed at the position and angle such that light beam B emitted upward is reflected off the lower surface of the upper portion of cylinder member 210. Light receiving element 124 is disposed at the position and angle to receive light beam B reflected off the lower surface of the upper portion of cylinder member 210.

Light emitting element 123 emits light beam B at a wavelength which is easily absorbed and scattered by liquid L and is not easily absorbed and scattered by air A. Light receiving element 124 detects the intensity of light beam B received thereby in analog form. Computer unit 130 of the chemical liquid injector of the present embodiment compares the intensity of light beam B detected by light receiving element 124 with a predetermined upper limit and determines the occurrence of abnormality when the intensity of light beam B excesses the upper limit.

### [Operation of Embodiment 2]

In the configuration described above, in air detection unit 400 of the present embodiment, light beam B emitted from light emitting element 123 is reflected off the lower surface of the upper portion of cylinder member 210, and the intensity of reflected light beam B is detected by light receiving element 124. light beam B is easily absorbed and scattered by liquid L and not easily absorbed and scattered by air A. As shown in Fig. 9(a), when cylinder member 210 is filled with liquid L, the intensity of light beam B detected by light receiving element 124 is below the predetermined upper limit.

When air A is trapped or filled into cylinder member 210 as shown in Figs. 9(b) and 9(c), the absorption and scattering of light beam B is reduced. This increases the intensity of light beam B detected by light receiving element 124 above the predetermined upper limit, resulting in the occurrence of abnormality determined by computer unit 130.

### [Modifications of Embodiment 2]

The present invention is not in any way limited to the abovementioned embodiment, but various changes or modifications may be made without departing from the scope of the invention. For example, air detection unit 400 of the same configuration as that in Embodiment 2 may be formed in a smaller size and disposed on the outer surface of extension tube 230 (not shown).

In the above-described embodiment, the wave emitting element comprises light emitting element 123 for emitting light beam B as a wave, while the wave detecting element comprises light receiving element 124 for detecting light beam B as a wave. For example, it is possible that the wave emitting element comprises an ultrasonic vibrator for emitting an ultrasonic wave as a wave, while the wave detecting element comprises an ultrasonic detector for detecting the ultrasonic wave as a wave (not shown).

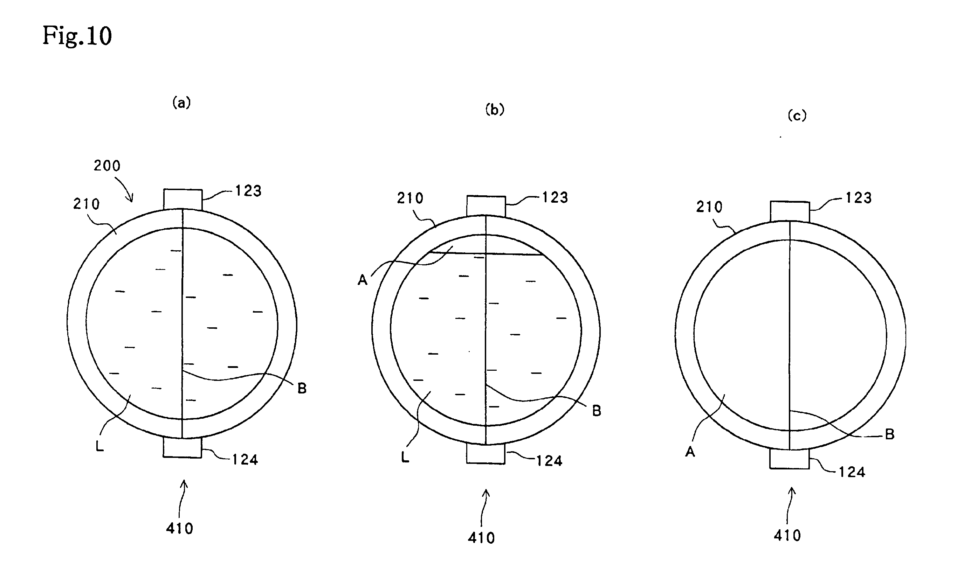

In the above-described embodiment, both of light emitting element 123 and light receiving element 124 are disposed below cylinder member 210, and light beam B emitted from light emitting element 123 and reflected off the inner surface of cylinder member 210 is detected by light receiving element 124. However, as shown in Figs. 10(a) to 10(c), it is possible that one of light emitting element 123 and light receiving element 124 is disposed below cylinder member 210 and the other is disposed above cylinder member 210, and light beam B emitted from light emitting element 123 and transmitted through cylinder member 210 is detected by light receiving element 124.

### [Configuration of Embodiment 3]

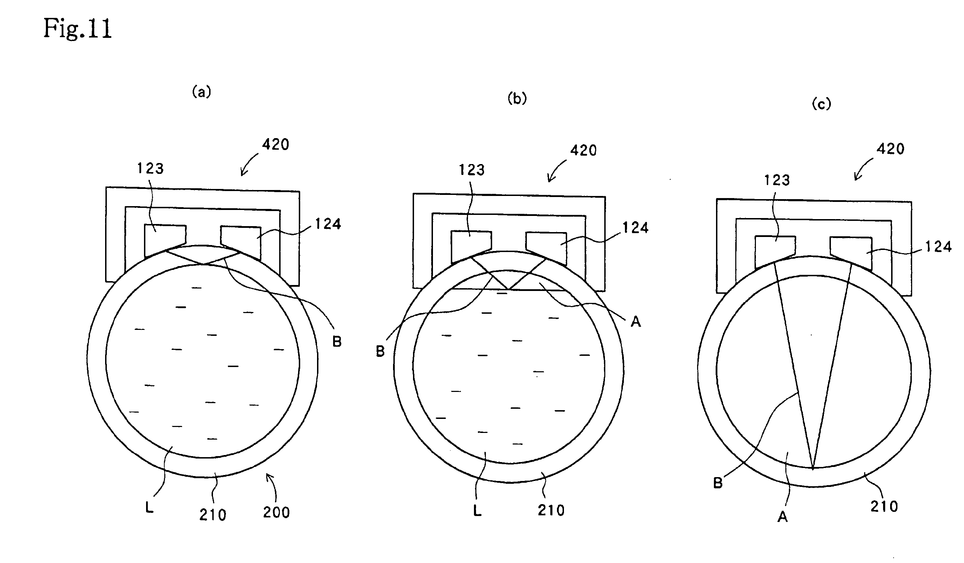

Next, Embodiment 3 of the present invention will hereinafter be described with reference to Figs. 11(a) to 11(c). In a chemical liquid injector (not shown) of the present embodiment, air detection unit 420 includes light emitting element 123 and light receiving element 124 just the same as Embodiment 1. Light emitting element 123 of air detection unit 420 emits light beam B as a wave downward to a predetermined position on the upper outer surface of cylinder member 210 at a predetermined angle. Light receiving element 124 detects light beam B entering it from below at a predetermined position on the upper outer surface of cylinder member 210.

Light emitting element 123 emits light beam B in pulses, and light receiving element 124 detects light beam B in pulses reflected off the surface of liquid L. Computer unit 130 measures the time period from the emission of light beam B from light emitting element 123 to the detection thereof by light receiving element 124, and determines the occurrence of abnormality if the measured time period does not comply with a predetermined allowable range.

### [Operation of Embodiment 3]

In the configuration described above, in air detection unit 420 of Embodiment 3, light beam B emitted in pulses from light emitting element 123 is reflected off the surface of liquid L and detected by light receiving element 124, and computer unit 130 measures the time period from the emission to the detection. The time period from the emission to the detection of light beam B is longer when air A is trapped or filled into cylinder member 210 as shown in Figs. 11(b) and 11(c) as compared with the case where cylinder member 210 is filled with liquid L as shown in Fig. 11(a). Thus, computer unit 130 can determine the occurrence of abnormality when the time period from the emission to the detection of light beam B does not comply with the predetermined allowable range.

### [Modifications of Embodiment 3]

The present invention is not in any way limited to the abovementioned embodiment, but various changes or modifications may be made without departing from the scope of the invention. For example, air detection unit 420 of the same configuration as that in Embodiment 3 may be formed in a smaller size and disposed on the outer surface of extension tube 230 (not shown).

In the above-described embodiment, the wave emitting element comprises light emitting element 123 for emitting light beam B as a wave, while the wave detecting element comprises light receiving element 124 for detecting light beam B as a wave. For example, it is possible that the wave emitting element comprises an ultrasonic vibrator for emitting an ultrasonic wave as a wave, while the wave detecting element comprises an ultrasonic detector for detecting the ultrasonic wave as a wave (not shown).

### [Configuration of Embodiment 4]

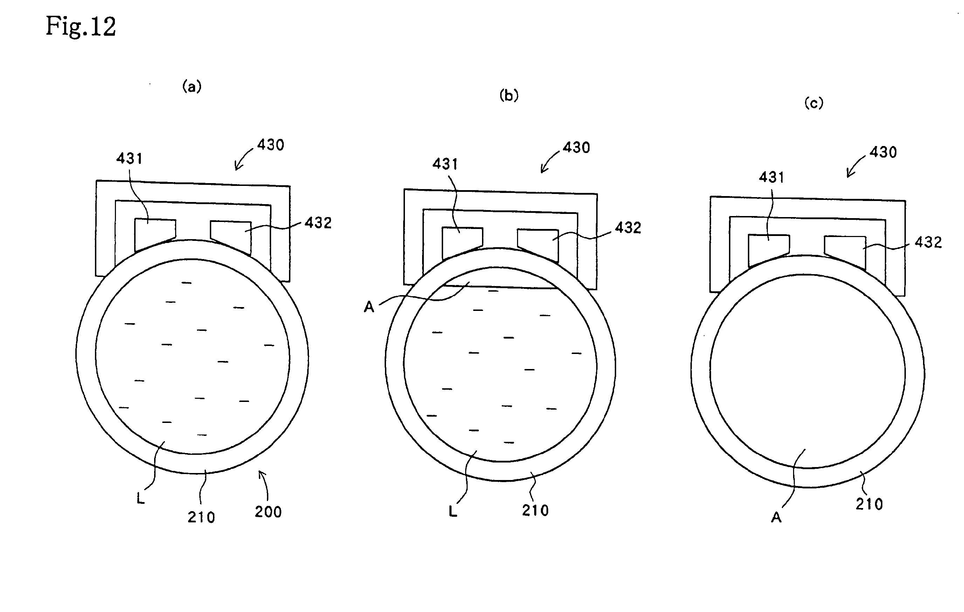

Next, Embodiment 4 of the present invention will hereinafter be described with reference to Figs. 12(a) to 12(c). In a chemical liquid injector (not shown) of the present embodiment, air detection unit 430 includes ultrasonic vibrator 431 and ultrasonic detector 432. Ultrasonic vibrator 431 emits ultrasonic wave S as a wave downward to a predetermined position on the upper outer surface of cylinder member 210 at a predetermined angle. Ultrasonic detector 432 detects ultrasonic wave S entering it from below at a predetermined position on the upper outer surface of cylinder member 210. Computer unit 130 analyzes the resonance characteristic of ultrasonic wave S detected by ultrasonic detector 432 and determines the occurrence of abnormality from the analyzed resonance characteristic.

### [Operation of Embodiment 4]

In the chemical liquid injector of Embodiment 4 with the abovementioned configuration, since ultrasonic wave S emitted from ultrasonic vibrator 431 of air detection unit 430 produces resonance of cylinder member 210 and liquid L and air A at different frequencies. The resonance characteristic of ultrasonic S detected by ultrasonic detector 432 can be analyzed by computer unit 130 to determine whether liquid L fills cylinder member 210 as shown in Fig. 12(a), air A is trapped in cylinder member 210 as shown in Fig. 12(b), or air A fills cylinder member 210 as shown in Fig. 12(c).

### [Modifications of Embodiment 4]

The present invention is not in any way limited to the abovementioned embodiment, but various changes or modifications may be made without departing from the scope of the invention. For example, air detection unit 430 of the same configuration as that in Embodiment 4 may be formed in a smaller size and disposed on the outer surface of extension tube 230 (not shown).

In the above-described embodiment, both of ultrasonic vibrator 431 and ultrasonic detector 432 are disposed above cylinder member 210, and ultrasonic wave S emitted from ultrasonic vibrator 431 and reflected off the inner surface of cylinder member 210 is detected by ultrasonic detector 432.

However, it is possible that both of ultrasonic vibrator 431 and ultrasonic detector 432 are disposed below cylinder member 210 (not shown), or that one of ultrasonic vibrator 431 and ultrasonic detector 432 is disposed below cylinder member 210 and the other is disposed above cylinder member 210, and ultrasonic wave S emitted from ultrasonic vibrator 431 and transmitted through the inner surface of cylinder member 210 is detected by ultrasonic detector 432 (not shown).

In above-described embodiment, the presence or absence of air A is determined by the analysis result of the resonance characteristic of ultrasonic wave S. For example, it is possible to determine the type of liquid L from the analyzed resonance characteristic. In this case, the determined type of liquid L may be notified as data display on touch panel 104, or the operation of piston driving mechanism 116 may be controlled in accordance with the determined type of liquid L.

In addition, it is possible to detect a foreign substance present in liquid L from the analysis result of the resonance characteristic of ultrasonic wave S. In this case, when a foreign substance is detected, the occurrence of abnormality may be determined to output a dedicated check alarm and to forcedly stop piston driving mechanism 116.

### [Configuration of Embodiment 5]

Next, Embodiment 5 of the present invention will hereinafter be described in brief. In a chemical liquid injector (not shown) of the present embodiment, an air detection unit (not shown) comprises a capacitance detecting sensor which detects a capacitance on the outer surface of cylinder member 210. Computer unit 130 compares the capacitance detected by the air detection unit with a predetermined allowable range, and determines the occurrence of abnormality when the capacitance does not comply with the allowable range.

### [Operation of Embodiment 5]

In the chemical liquid injector of Embodiment 5 with the abovementioned configuration, since the air detection unit detects the capacitance of cylinder member 210 of liquid syringe 200, the capacitance can be used to determine whether liquid L fills cylinder member 210, or air A is trapped or filled into cylinder member 210.

## Claims

1. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a wave emitting element for emitting a wave to a predetermined position on an outer surface of the held cylinder member at a predetermined angle;
a wave detecting element for detecting the intensity of the wave at a predetermined position on the outer surface of the held cylinder member;
abnormality determining means for determining occurrence of abnormality when the detected intensity of the wave does not comply with a predetermined allowable range; and
alarm notifying means for outputting a check alarm when the occurrence of abnormality is determined.

2. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a wave emitting element for emitting a wave to a predetermined position on an outer surface of an extension tube;
a wave detecting element for detecting the intensity of the wave at a predetermined position on the outer surface of the extension tube;
abnormality determining means for determining occurrence of abnormality when the detected intensity of the wave does not comply with a predetermined allowable range; and
alarm notifying means for outputting a check alarm when the occurrence of abnormality is determined.

3. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a wave emitting element for emitting a wave to a predetermined position on an outer surface of the held cylinder member at a predetermined angle;
a wave detecting element for detecting the wave at a predetermined position on the outer surface of the held cylinder member;
time measuring means for measuring a time period from the emission to the detection of the wave;
abnormality determining means for determining occurrence of abnormality when the measured time period does not comply with a predetermined allowable range; and
alarm notifying means for outputting a check alarm when the occurrence of abnormality is determined.

4. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a wave emitting element for emitting a wave to a predetermined position on an outer surface of an extension tube at a predetermined angle;
a wave detecting element for detecting the wave at a predetermined position on the outer surface of the extension tube;
time measuring means for measuring a time period from the emission to the detection of the wave;
abnormality determining means for determining occurrence of abnormality when the measured time period does not comply with a predetermined allowable range; and
alarm notifying means for outputting a check alarm when the occurrence of abnormality is determined.

5. A chemical liquid injector according to any one of claims 1 to 4, wherein said wave emitting element emits light beam as the wave, and
said wave detecting element detects light beam as the wave.

6. A chemical liquid injector according to any one of claims 1 to 4, wherein said wave emitting element emits an ultrasonic wave as the wave, and
said wave detecting element detects an ultrasonic wave as the wave.

7. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a wave emitting element for emitting light beam to a predetermined position on an outer surface of the held cylinder member at a predetermined angle;
a wave detecting element for detecting the light beam at a predetermined position on the outer surface of the held cylinder member;
abnormality determining means for determining occurrence of abnormality when the light beam is detected; and
alarm notifying means for outputting a check alarm when the occurrence of abnormality is determined.

8. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a wave emitting element for emitting light beam to a predetermined position on an outer surface of a extension tube at a predetermined angle;
a wave detecting element for detecting the light beam at a predetermined position on the outer surface of the extension tube;
abnormality determining means for determining occurrence of abnormality when the light beam is detected; and
alarm notifying means for outputting a check alarm when the occurrence of abnormality is determined.

9. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a wave emitting element for emitting an ultrasonic wave to a predetermined position on an outer surface of the held cylinder member at a predetermined angle;
a wave detecting element for detecting the ultrasonic wave at a predetermined position on the outer surface of the held cylinder member;
characteristic analyzing means for analyzing a resonance characteristic of the detected ultrasonic wave;
abnormality determining means for determining occurrence of abnormality from the analyzed resonance characteristic; and
alarm notifying means for outputting a check alarm when the occurrence of abnormality is determined.

10. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a wave emitting element for emitting an ultrasonic wave to a predetermined position on an outer surface of an extension tube at a predetermined angle;
a wave detecting element for detecting the ultrasonic wave at a predetermined position on the outer surface of the extension tube;
characteristic analyzing means for analyzing a resonance characteristic of the detected ultrasonic wave;
abnormality determining means for determining occurrence of abnormality from the analyzed resonance characteristic; and
alarm notifying means for outputting a check alarm when the occurrence of abnormality is determined.

11. A chemical liquid injector according to claim 9 or 10, further comprising:
liquid determining means for the type of the liquid from the analyzed resonance characteristic; and
liquid outputting means for outputting the determined type of the liquid.

12. A chemical liquid injector according to any one of claims 9 to 11, further comprising:
liquid determining means for the type of the liquid from the analyzed resonance characteristic; and
injection control means for controlling the operation of the piston driving mechanism based on the determined type of the liquid.

13. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a capacitance detecting sensor for detecting a capacitance on an outer surface of the held cylinder member;
abnormality determining means for determining occurrence of abnormality when the detected capacitance does not comply with a predetermined allowable range; and
alarm notifying means for outputting a check alarm when the occurrence of abnormality is determined.

14. A chemical liquid injector for injecting a chemical liquid from a liquid syringe including a cylinder member and a piston member slidably inserted into the cylinder member into a patient through an extension tube, comprising:
a cylinder holding mechanism for removably holding a cylinder member of a liquid syringe;
a piston driving mechanism for moving a piston member relative to the held cylinder member;
a capacitance detecting sensor for detecting a capacitance on an outer surface of an extension tube;
abnormality determining means for determining occurrence of abnormality when the detected capacitance does not comply with a predetermined allowable range; and
alarm notifying means for outputting a check alarm when the occurrence of abnormality is determined.

15. A chemical liquid injector according to any one of claims 1, 3, 7, 9, and 13, wherein the liquid syringe includes a plurality of types different from each other in at least one of the cylinder member and the liquid,
the chemical liquid injector further comprising type inputting means for inputting data of at least the type of the liquid syringe,
wherein the abnormality determining means determines the occurrence of abnormality based on the type.

16. A chemical liquid injector according to any one of claims 1, 3, 7, and 9, wherein the liquid syringe includes a plurality of types different from each other in the outer diameter of the cylinder member,
the chemical liquid injector further comprising component placing mechanism for movably supporting said wave emitting element and said wave detecting element to place them on the outer surface of the cylinder member.

17. A chemical liquid injector according to claim 16, wherein said component placing mechanism places at least one of said wave emitting element and said wave detecting element on the outer surface of an upper portion of the cylinder member.

18. A chemical liquid injector according to claim 16, wherein said component placing mechanism places at least one of said wave emitting element and said wave detecting element on the outer surface of a lower portion of the cylinder member.

19. A chemical liquid injector according to any one of claims 16 to 18, wherein said component placing mechanism places said wave emitting element and said wave detecting element near the leading end of the cylinder member.

20. A chemical liquid injector according to 13, wherein the liquid syringe includes a plurality of types different from each other in the outer diameter of the cylinder member,
the chemical liquid injector further comprising a component placing mechanism for movably supporting said capacitance detecting sensor to place it on the outer surface of the cylinder member.

21. A chemical liquid injector according to any one of claims 1 to 20, further comprising a drive stopping means for forcedly stopping said piston driving mechanism when the occurrence of abnormality is determined.

22. A chemical liquid injector according to any one of claims 1 to 21, further comprising a stop outputting means for outputting a stop signal to the outside when the occurrence of abnormality is determined.

23. A chemical liquid injecting system for imaging an image of a patient by a diagnostic imaging apparatus, the patient being injected with at least a contrast media by a chemical liquid injector, comprising:
a chemical liquid injector according to claim 22,
wherein a diagnostic imaging apparatus comprises imaging means for taking an image, stop inputting means for receiving the stop signal from said chemical liquid injector, and alarm outputting means for outputting a check alarm when the stop signal is input thereto from the outside.

24. A chemical liquid injecting system according to claim 23, wherein said diagnostic imaging apparatus further comprises imaging stopping means for forcedly stopping the imaging operation of the imaging means when the stop signal is input thereto from the outside.
